Europäisches Patentamt

(19) European Patent Office

Office européen des brevets

(11) Numéro de publication : **0 307 289 B1**

(12) **FASCICULE DE BREVET EUROPEEN**

(45) Date de publication du fascicule du brevet :
**27.11.91 Bulletin 91/48**

(51) Int. Cl.$^5$ : **G01N 15/08, G01N 33/36**

(21) Numéro de dépôt : **88402211.2**

(22) Date de dépôt : **02.09.88**

(54) **Procédé et dispositif de mesure de la perméabilité à l'air de matériaux poreux en défilement continu.**

(30) Priorité : **08.09.87 FR 8712652**

(43) Date de publication de la demande :
**15.03.89 Bulletin 89/11**

(45) Mention de la délivrance du brevet :
**27.11.91 Bulletin 91/48**

(84) Etats contractants désignés :
**DE GB**

(56) Documents cités :
**EP-A- 0 096 311**
**EP-A- 0 107 482**
**WO-A-87/02771**
**US-A- 3 466 925**

(73) Titulaire : **INSTITUT TEXTILE DE FRANCE**
**35, rue des Abondances, B.P. 79**
**F-92105 Boulogne-Billancourt Cédex (FR)**
Titulaire : **FYLTIS**
**89, rue de la Villette (BP 3175)**
**F-69211 Lyon Cédex 03 (FR)**

(72) Inventeur : **Payot, Jean-Marie**
**5, rue de la Tour du Pin**
**F-69004 Lyon (FR)**

(74) Mandataire : **Descourtieux, Philippe et al**
**CABINET BEAU de LOMENIE 55 rue**
**d'Amsterdam**
**F-75008 Paris (FR)**

**Description**

La présente invention concerne la mesure de la perméabilité à l'air sur des matériaux poreux et en particulier sur des étoffes textiles en déplacement continu. Elle concerne notamment un dispositif spécialement adapté pour une telle mesure.

La perméabilité à l'air est une caractéristique physique qu'il est indispensable de connaître pour certains matériaux dont l'usage est lié à leur porosité ; il en est ainsi pour les matériaux utilisés comme filtres. Le choix de la contexture et des différents composants constitutifs du matériau filtrant est conditionné par les conditions de sa mise en oeuvre. Mais pour une même contexture de base et les mêmes composants, il importe pour le fabricant de contrôler l'homogénéité de son produit tout au long de sa fabrication. Un moyen d'effectuer ce contrôle est de réaliser des prélèvements sur l'étoffe et de mesurer en statique la perméabilité à l'air des échantillons prélevés. Une autre voie, utilisée dans l'industrie papetière, est d'effectuer la mesure sur le papier en défilement continu en faisant passer celui-ci sur une rampe rectangulaire d'aspiration sur toute la largeur du papier et en mesurant dans le conduit d'aspiration de la rampe le débit massique de l'air aspiré. Une telle méthode est décrite dans US-A-3466925. Cette méthode présente deux inconvénients : elle est globale et ne rend pas compte des variations localisées de perméabilité, deux différences de porosité en sens opposé sur la largeur du papier pouvant se compenser ; elle n'est pas utilisable pour les étoffes rigides et lisses compte-tenu des fuites qui se produisent entre l'étoffe et la rampe d'aspiration.

Or on a trouvé, et c'est ce qui fait l'objet de l'invention, un procédé de mesure de la perméabilité à l'air de matériaux poreux en défilement continu qui pallie les inconvénients constatés. Le procédé selon l'invention est défini par la revendication 1.

Ainsi l'incurvation du matériau à l'intérieur de la bouche d'aspiration n'est pas liée aux forces d'aspiration mises en oeuvre ni à la rigidité du matériau poreux, elle est créée par des moyens extérieurs d'application. De préférence l'incurvation du matériau poreux vers l'intérieur de la bouche d'aspiration est constante pour un même matériau poreux. L'incurvation constante est obtenue en faisant passer le matériau poreux dans la zone correspondant à l'axe de symétrie de la bouche d'aspiration et du conduit d'aspiration sous une surface de déviation située en dessous du plan du matériau en défilement sur la bouche d'aspiration. Avantageusement, l'incurvation est réglable en faisant varier la position relative de la surface de déviation par rapport au plan du matériau en défilement.

Ainsi du fait de la forme circulaire de la bouche d'aspiration et de l'incurvation contrôlée du matériau, par des moyens extérieurs, le débit massique mesuré correspond entièrement à l'air passant à travers le matériau poreux, les risques de fuite ayant été supprimés.

C'est un autre objet de l'invention que de proposer un dispositif spécialement conçu pour mettre en oeuvre le procédé précité. Ce dispositif de mesure de la perméabilité à l'air d'un matériau poreux en défilement continu est décrit par la revendication 4.

Les moyens d'application consistent par exemple en une surface de déviation prenant appui sur le matériau poreux dans la zone correspondant à l'axe de symétrie de la bouche d'aspiration et du conduit d'aspiration, ladite surface étant située sous le plan du matériau en défilement sur la bouche d'aspiration. Avantageusement, la surface de déviation, prenant appui au-dessus du matériau poreux, consiste en une bille sphérique, mobile en rotation à l'intérieur d'un logement cylindrique coaxial à la bouche et au conduit d'aspiration et assortie d'une surface de glissement située au droit de la bille sous le matériau poreux.

Le réglage de l'incurvation du matériau poreux est obtenu en en faisant varier la hauteur de la surface de glissement suivant l'axe de symétrie de la bouche et du conduit d'aspiration.

L'utilisation d'une bille sphérique comme surface de déviation présente des avantages. La bille prend appui sur le matériau et incurve celui-ci sous l'effet de son poids ; cependant étant libre en rotation, la bille tourne dans son logement cylindrique pendant le déplacement du matériau ; le seul glissement provenant de la déviation se situe au niveau de la surface inférieure de glissement qui règle en hauteur la position de la bille et le matériau constitutif de ladite surface de glissement est poli de telle sorte qu'il n'y ait aucune altération du matériau poreux due au passage dans le dispositif de mesure.

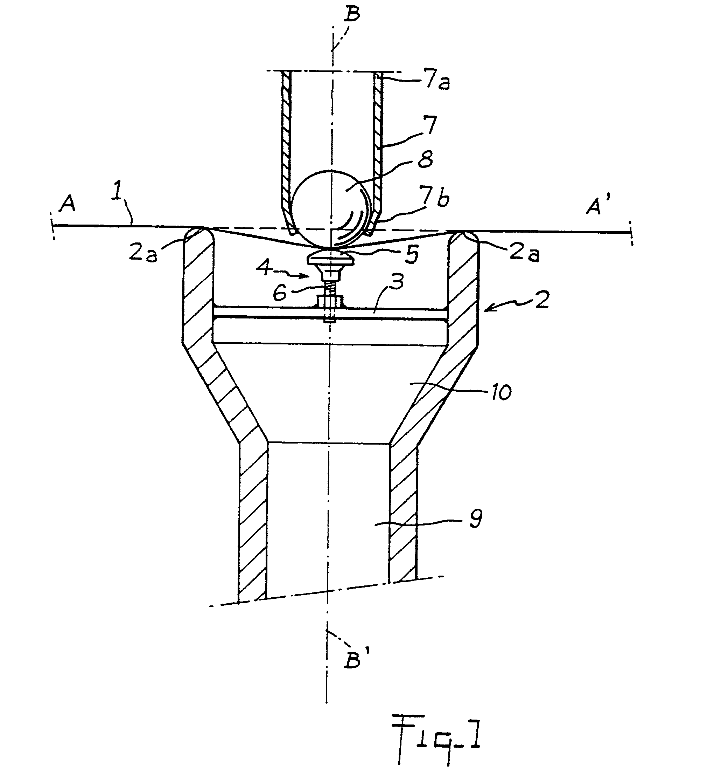

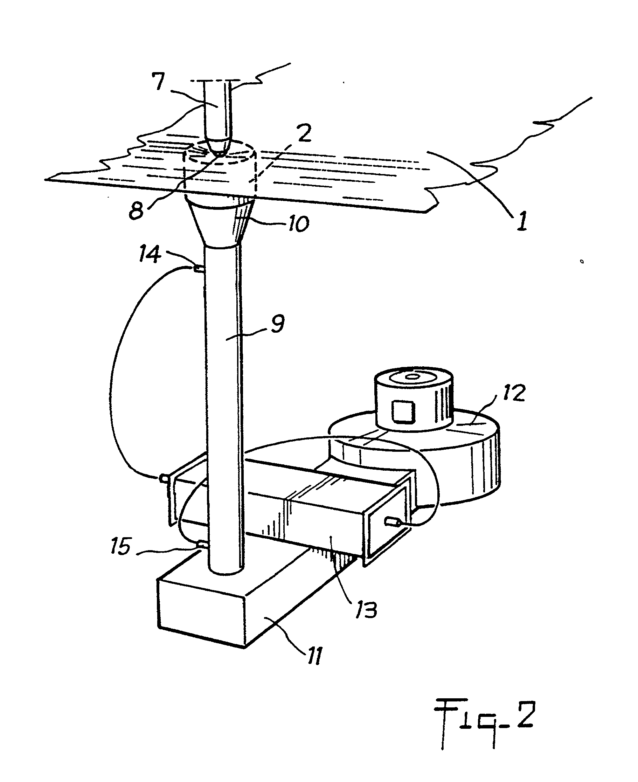

L'invention sera mieux comprise à la lecture de la description qui va maintenant être faite du mode préféré de réalisation illustrée par le dessin annexé dans lequel : la figure 1 est une vue en coupe schématique de la bouche d'aspiration et de la surface de déviation et la figure 2 est une vue en perspective de l'ensemble du dispositif.

Le dispositif de mesure de la perméabilité à l'air est placé dans une chaîne soit de production soit de contrôle du matériau poreux, à travers laquelle ledit matériau se déplace de façon continue et à vitesse constante. Le dispositif est positionné de telle sorte que le parcours du matériau poreux 1 coïncide avec le plan AA' correspondant à la surface supérieure de la bouche d'aspiration 2. Celle-ci est un cylindre dont l'axe de symétrie BB' est perpendiculaire au plan AA'. Le bord supérieur 2a de la bouche d'aspiration 2, en contact avec le matériau 1, est arrondi de manière à réduire le coefficient de frottement.

A l'intérieur de la bouche 2 et montée selon un diamètre de celle-ci, la tige 3 supporte la pièce 4 comportant à sa partie supérieure une plaque 5 polie et à sa partie inférieure une tige filetée 6 coopérant avec le filetage pratiqué dans la tige 3 au droit de l'axe BB'. Ainsi la plaque 5 est réglable en hauteur et est située dans la zone correspondant à l'axe de symétrie BB' de la bouche d'aspiration 2.

Au-dessus de la bouche 2, le logement 7 est dans sa partie supérieure un tube cylindrique dont le diamètre interne permet le passage de la bille 8 et dans sa partie inférieure 7b le tube prend une forme légèrement conique dont le diamètre interne est légèrement inférieur au diamètre de la bille 8. Ainsi la bille 8 est introduite dans le logement, et elle peut y tourner librement tout en étant maintenue au niveau 7b du logement 7.

La bille 8 est une bille d'acier de diamètre 25 mm. Le logement 7 a une hauteur de 200 mm et un diamètre interne de 26 mm dans la partie supérieure 7a et de 24 mm dans la partie inférieure 7b. La bouche d'aspiration 2 a un diamètre intérieur de 80 mm.

Le conduit 9 d'aspiration a un diamètre (30 mm) inférieur à celui de la bouche 2 d'aspiration. Il est relié à la bouche 2 d'aspiration par un tube de venturi 10 ayant une conicité de 7° d'angle.

Le conduit cylindrique d'aspiration 9 débouche dans un conduit 11 de section rectangulaire en bout duquel est disposée une turbine 12 assurant la dépression créant l'aspiration. Le capteur 13 est du type connu, il mesure le débit massique de l'air de déplacement dans le conduit 9 entre les deux emplacements 14 et 15. Le capteur 13 est maintenu horizontal pour éviter la création de micro-courants ascentionnels qui rendraient difficiles le réglage du zéro.

Le fonctionnement du dispositif de mesure est le suivant. Le logement 7 étant dans une position haute, on introduit le matériau poreux 1 dont on veut mesurer la perméabilité à l'air au-dessus de la bouche d'aspiration 2 selon le plan AA'. Cette introduction étant faite, on descend le logement 7, contenant la bille 8, de sorte que la bille 8 prend appui sur le matériau 1 et l'incurve jusqu'à ce que la surface inférieure dudit matériau 1 soit en contact avec le dessus de la plaque 5. Dans cette ultime position, la bille 8 tourne librement à l'intérieur du logement 7 sans frottement intensif avec les parois du cône 7b. Puis on met en fonctionnement la turbine 12 et on mesure le débit massique à l'aide du capteur 13, pendant le déplacement du matériau 1.

L'arrondi du bord 2a interne de la bouche 2 et la déviation provoquée par la bille permettent d'obtenir une étanchéité parfaite quel que soit le matériau, y compris des matériaux rigides.

A titre d'exemple, on a mesuré la perméabilité à l'air d'une étoffe de 80 mètres de long et de 960 mm de large se déplaçant à une vitesse constante de 4,1 mètres par minute. La bouche 2 était placée à 300 mm de la lisière. L'enregistrement de la mesure a été fait, la pièce se déplaçant dans un sens, puis dans l'autre;

ainsi on a effectué les mesures selon les deux lisières de l'étoffe. Les mesures dans ce sens aller ont donné une perméabilité à l'air de 1960 m³/h/m² avec une variation de 100 m³/h · m² soit une variation de 5%. Dans le sens retour, les mesures ont donné une perméabilité de 2.200 m³/h · m² avec une variation de 150 m³/h · m² soit une variation de 7%. La différence de perméabilité d'un côté à l'autre de l'étoffe est de 240 m³/h · m². La dépression était de 10 mm de colonne d'eau.

Par ailleurs, l'examen attentif des mesures de débit massique permet éventuellement de déceler des anomalies dans la chaîne de production du matériau. Ainsi des fluctuations périodiques du débit sont des indicateurs d'un dysfonctionnement auquel il importe de remédier, par exemple excentration du cylindre de calandrage qui modifie localement l'écrasement de l'étoffe et donc modifie selon la même périodicité sa perméabilité.

**Revendications**

1. Procédé de mesure de la perméabilité à l'air d'un matériau poreux en défilement continu consistant à appliquer une petite portion de la surface de ce matériau poreux (1) sur une bouche d'aspiration (2) reliée à des moyens d'aspiration (12) par un conduit cylindrique (9) coaxial à la bouche d'aspiration (2) et à mesurer le débit massique de l'air aspiré par le conduit d'aspiration (9) caractérisé en ce que, la bouche d'aspiration (2) étant de forme circulaire et ayant un bord interne (2a) arrondi, le matériau est constamment et uniformément incurvé vers l'intérieur de la bouche d'aspiration (2).

2. Procédé selon la revendication 1 caractérisé en ce que l'incurvation est obtenue en faisant passer le matériau poreux (1) dans la zone correspondant à l'axe de symétrie (BB') de la bouche d'aspiration (2) et du conduit d'aspiration (9) sous une surface de déviation (8) située en dessous du plan du matériau en défilement sur la bouche d'aspiration.

3. Procédé selon la revendication 2 caractérisé en ce qu'on règle l'incurvation du matériau poreux (1) vers l'intérieur de la bouche d'aspiration (2) en faisant varier la position relative de la surface de déviation (8) par rapport au plan (AA') du matériau en défilement.

4. Dispositif de mesure de la perméabilité à l'air d'un matériau poreux en défilement continu comprenant une bouche d'aspiration (2) reliée à des moyens d'aspiration (12) par un conduit cylindrique (9) coaxial à la bouche d'aspiration (2) et des moyens de mesure (13, 14, 15) du débit massique de l'air aspiré dans le conduit d'aspiration (9) caractérisé en ce que la bouche d'aspiration (2) est circulaire et a un bord interne (2a) arrondi et en ce qu'il comprend des moyens extérieurs d'application (8, 4) agencés de telle sorte qu'ils appliquent le matériau (1) vers l'intérieur de la bouche d'aspiration (2), moyennant quoi ledit matériau (1) est incurvé vers l'intérieur de la bouche d'aspiration (2) pendant son défilement continu.

5. Dispositif selon la revendication 4 caractérisé en ce que les moyens extérieurs d'application consistent en une surface (8) de déviation prenant appui sur le matériau poreux (1) dans la zone correspondant à l'axe de symétrie (BB') de la bouche d'aspiration (2) et du conduit d'aspiration (9), ladite surface (8) étant située sous le plan (AA') du matériau (1) en défilement sur la bouche d'aspiration (2).

6. Dispositif selon la revendication 5 caractérisé en ce que la surface de déviation consiste en une bille (8) sphérique, mobile en rotation à l'intérieur d'un logement (7) cylindrique coaxial à la bouche (2) et au conduit (9) d'aspiration et assortie d'une surface (5) de glissement située au droit de la bille (8) sous le matériau poreux (1).

7. Dispositif selon la revendication 6 caractérisé en ce que la surface de glissement (5) est réglable en hauteur le long de l'axe de symétrie (BB') de la bouche (2) et du conduit (9) d'aspiration.

## Patentansprüche

1. Verfahren zum Messen der Luftdurchlässigkeit eines porösen Materials, das sich kontinuierlich bewegt, bestehend aus Anbringen eines kleinen Bereichs der Oberfläche dieses porösen Materials (1) auf einer Absaugmündung (2), die mit einer Absaugeinrichtung (12) über eine zylindrische Leitung (9) verbunden ist, die koaxial zur Absaugmündung (2) sich erstreckt und Messen der Massenrate der angesaugten Luft durch den Absaugkanal (9), **dadurch gekennzeichnet**, daß die Absaugmündung (2) eine Kreisform und einen inneren abgerundeten Rand (2a) aufweist, wobei das Material kontinuierlich und gleichmäßig nach dem Inneren der Absaugmündung (2) gebogen ist.

2. Verfahren nach Anspruch 1, **dadurch gekennzeichnet**, daß die Biegung unter Durchlassen des porösen Materials (1) in der entsprechenden Zone mit der Symmetrieachse (B, B') der Absaugmündung (2) und der Absaugleitung (9) erhalten wird, unter einer Ablenkoberfläche (8), die unterhalb der Ebene des sich vorbeibewegenden Materials auf der Absaugmündung angeordnet ist.

3. Verfahren nach Anspruch 2, **dadurch gekennzeichnet**, daß die Krümmung des porösen Materials (1) nach dem Inneren der Absaugmündung (2) eingestellt wird durch Verändern der relativen Stellung der Ablenkoberfläche (8) bezüglich der Ebene (A, A') des vorbeiziehenden Materials.

4. Vorrichtung zum Messen der Luftdurchlässigkeit eines porösen, sich kontinuierlich vorbeibewegenden Materials mit einer Absaugmündung (2), die mit Absaugeinrichtungen (12) durch eine Absaugleitung (9) verbunden ist, und Meßeinrichtungen (13, 14, 15) der Massenrate der in die Absaugleitung (9) angesaugten Luft aufweist, **dadurch gekennzeichnet**, daß die Ansaugmündung (2) kreisförmig ist und einen inneren abgerundeten Rand (2a) aufweist, und daß sie äußere Aufbringeinrichtungen (8, 4) aufweist, die derart ausgebildet sind, daß sie das Material (1) nach dem Inneren der Absaugmündung (2) beaufschlagen, so daß das Material (1) nach dem Inneren der Absaugmündung (2) während seiner kontinuierlichen Bewegung gekrümmt wird.

5. Vorrichtung nach Anspruch 4, **dadurch gekennzeichnet**, daß die äußeren Aufbringeinrichtungen bestehen aus einer Ablenkoberfläche (8), die gegen das poröse Material (1) in der Zone anliegt, die der Symmetrieachse (B, B') der Absaugmündung (2) und der Absaugleitung (9) entspricht, wobei die Oberfläche (8) unter der Ebene (A, A') des auf der Absaugmündung (2) vorbeiziehenden Materials (1) angeordnet ist.

6. Vorrichtung nach Anspruch 5, **dadurch gekennzeichnet**, daß die Ablenkoberfläche aus einer Kugel (8) besteht, die drehbweglich im Inneren einer zylindrischen Aufnahme (7) bewegbar ist, die koaxial zur Mündung (2) und zur Absaugleitung (9) ist und mit einer Gleitoberfläche (5) versehen ist, die im Bereich der Kugel (8) unter dem porösen Material (1) angeordnet ist.

7. Vorrichtung nach Anspruch 6, **dadurch gekennzeichnet**, daß die Gleitoberfläche (5) in der Höhe entlang der Symmetrieachse (B, B') der Mündung (2) und der Absaugleitung (9) einstellbar ist.

## Claims

1. A method of measuring the air-permeability of a continuously moving porous material, comprising applying a small portion of the surface of said porous material (1) against a suction nozzle (2) connected to suction means (12) via a cylindrical conduit (9) coaxial with the suction nozzle (2) and measuring the mass rate of flow of air sucked in by the suction conduit (9), characterised in that the suction nozzle (2) is of a circular shape with a rounded inner edge (2a) and the material is constantly and uniformly curved towards the interior of the suction nozzle (2).

2. A method according to claim 1, characterised in that the curving is produced by passing the porous material (1), in the zone corresponding to the axis of symmetry (BB') of the suction nozzle (2) and of the suction conduit (9), beneath a deflecting surface (8) situated below the plane of the material as it moves over the suction nozzle.

3. A method according to claim 2, characterised in that the curving of the porous material (1) towards the interior of the suction nozzle (2) is controlled by varying the relative position of the deflecting surface (8) with respect to the plane (AA') of the moving material.

4. Apparatus for measuring the air permeability of a continuously moving porous material comprising a suction nozzle (2) connected to suction means (12) by a cylindrical conduit (9) coaxial to the suction nozzle (2) and means (13, 14, 15) for measuring the mass rate of flow of the air sucked into the suction conduit (9), characterised in that the suction nozzle (2) is circular and has a rounded inner edge (2a) and in that it comprises external application means (8, 4) so arranged that they apply the material (1) to the interior of the suction nozzle (2), so that said material (1) is curved towards the interior of the suction nozzle (2) during its continuous movement.

5. Apparatus according to claim 4, characterised in that the external application means comprise a deflection surface (8) bearing on the porous material (1) in the zone corresponding to the axis of symmetry (BB') of the suction nozzle (2) and of the suction conduit (9), said surface (8) being situated beneath the plane (AA') of the material (1) moving over the suction nozzle (2).

6. Apparatus according to claim 5, characterised in that the deflecting surface consists of a spherical ball (8) which is rotatable inside a cylindrical housing (7) coaxial with the suction nozzle (2) and with the suction conduit (9) and associated with a slideway surface (5) situated level with the ball (8) beneath the porous material (1).

7. Apparatus according to claim 6, characterised in that the slideway surface (5) is vertically adjustable along the axis of symmetry (BB') of the suction nozzle (2) and conduit (9).

Fig-1

Fig-2